# EUROPÄISCHE PATENTSCHRIFT

(11) **EP 1 565 221 B1**
(45) Veröffentlichungstag und Bekanntmachung des Hinweises auf die Patenterteilung: **29.11.2006**
(21) Anmeldenummer: 03812139.8
(22) Anmeldetag: 29.10.2003
(51) Int. Cl.: A61M 5/158, A61M 39/26, A61M 25/02

(54) **Katheterkopf mit verschließbarem Dichtelement**
Catheter head with closing seal element
Tête de cathéter munie d'un élément d'étanchéité obturable

(30) Priorität: 29.11.2002 DE 10255817
(43) Veröffentlichungstag der Anmeldung: 24.08.2005
(73) Patentinhaber: Disetronic Licensing AG, 3401 Burgdorf (CH)
(72) Erfinder: BÜTIKOFER, Markus, 3110 Münsingen (CH); ZIHLMANN, Rudolf, CH-3550 Langnau (CH); SCHEURER, Simon, CH-3006 Bern (CH)
(74) Vertreter: Küng, Peter
(86) Internationale Anmeldenummer: PCT/EP2003/012003
(87) Internationale Veröffentlichungsnummer: WO 2004/050148

(56) Entgegenhaltungen:
- EP-A- 0 704 227
- EP-A- 1 016 431
- WO-A-02/081012
- US-A- 5 522 803
- US-A- 5 968 011
- US-A1- 2002 161 332

## Beschreibung

Die Erfindung betrifft einen Katheterkopf für medizinische, vorzugsweise therapeutische, und pharmazeutische Anwendungen. Der Katheterkopf kann insbesondere Teil eines Infusionssets sein oder zusammen mit einem Katheter und/oder einem oder mehreren anderen fluidführenden Elementen ein Infusionsset bilden.

Ein Katheterkopf, wie er beispielsweise aus der DE 198 21 723 C bekannt ist, besteht aus einem Grundkörper, der auf organischem Gewebe, beispielsweise auf der menschlichen Haut, positionierbar ist, und einem Verbindungskörper, der einen der Fluidzuführung dienenden Katheter mit dem Grundkörper verbindet. Von einer Unterseite des Grundkörpers oder seitlich ragt eine Kanüle ab, die in das Gewebe eingeführt und durch die Positionierung des Grundkörpers auf dem Gewebe im eingeführten Zustand fixiert wird. Der Grundkörper dient sozusagen als Anschluss für die Kanüle. In ein Gehäuse des Grundkörpers ist ein Septum eingelassen, das die Kanüle hermetisch nach außen abdichtet. Der Verbindungskörper umfasst eine Verbindungsnadel, die über einen internen Fluidkanal des Verbindungskörpers mit dem Katheter oder anderen fluidführenden Teilen eines Infusionssystems verbunden ist. Wird der Verbindungskörper mit dem Grundkörper verbunden, durchsticht die Verbindungsnadel das Septum, so dass der interne Fluidkanal des Verbindungskörpers durch die Verbindungsnadel und einen internen Fluidkanal des Grundkörpers mit der Kanüle fluidisch verbunden ist. Der Verbindungskörper kann von dem Grundkörper getrennt und wiederholt mit dem Grundkörper verbunden werden. Bei jedem Herstellen der Verbindung durchsticht die Verbindungsnadel das Septum. Das Septum hat die Eigenschaft, dass es die Kanüle stets hermetisch nach außen abdichtet, wenn die Verbindungsnadel bei dem Lösen der Verbindung zwischen dem Grundkörper und dem Verbindungskörper aus dem Septum herausgezogen worden ist.

Das Patent US 5,968,011 offenbart einen Katheterkopf mit einem Deckelelement, einer Kanüle und einem Septum, welches eine Kavität von der Aussenwelt abdichtet. Das Deckelelement ist relativ zur Kanüle des Katheterkopfes und das Septum um eine Zentralachse der Kanüle drehbar gelagert (siehe Figur 7). Das Deckelelement weist eine Öffnung auf (siehe Figur 16). Durch Rotation des Deckelements um die Zentralachse der Kanüle kann seine Öffnung mit dem Flüssigkeitspfad des Katheterkopfs ausgerichtet werden, so dass der Flüssigkeitspfad durchgängig ist und Flüssigkeit durch die Kanüle ins Gewebe infundiert werden kann bzw. der Flüssigkeitspfad unterbrochen ist. Die Anschlussseite des Septums, über die der Flüssigkeitspfad mit der Kanüle des Katheterkopfes verbindbar ist, ist gegenüber der Kanüle abgewinkelt.

Es ist eine Aufgabe der Erfindung, bei einem Katheterkopf für medizinische und pharmazeutische Anwendungen die Abdichtung einer Kanüle zu verbessern, die im Gebrauch in ein Gewebe eingeführt ist.

Die Erfindung betrifft einen Katheterkopf für medizinische und pharmazeutische Anwendungen gemäß den Ansprüchen. Bevorzugt wird er in therapeutischen Anwendungen für die Verabreichung eines fluiden Produkts, beispielsweise einer Infusionsflüssigkeit, verwendet. Ein prominentes Beispiels solch einer Infusionsflüssigkeit ist Insulin in der Diabetestherapie. Grundsätzlich kann der Katheterkopf jedoch auch für diagnostische Anwendungen eingesetzt werden, um organischem Gewebe Flüssigkeit zu entziehen und dein jeweiligen Diagnosezweck entsprechend zu analysieren.

Der Katheterkopf umfasst einen auf organischem Gewebe, vorzugsweise der menschlichen Haut, positionierbaren Grundkörper und einen Verbindungskörper, der mit dem Grundkörper lösbar verbünden ist oder erst noch verbunden werden kann. Der Verbindungskörper ist mit einem Katheter oder einem anderen, für die Fluidführung geeigneten Element verbunden und bildet eine Fluidverbindung mit wenigstens einem Fluidkanal, durch den ein Fluid, insbesondere eine Flüssigkeit, von dem Katheter oder dem anderen für die Fluidführung geeigneten Element zu dem Grundkörper oder in die umgekehrte Richtung strömen kann. Der Verbindungskörper dient somit als Fluidanschluss für den Grundkörper. Der Grundkörper, der Verbindungskörper und ein mit dem Verbindungskörper verbundener Katheter mit einem üblichen Anschlussende bilden vorzugsweise bereits ein Infusionsset, das als solches in den Handel gelangt.

Der Begriff "Körper" soll ausdrücken, dass die derart bezeichneten Teile wie ein einziges Teil handhabbar sind. Die als "Körper" bezeichneten Einheiten können zwar einstückig sein, müssen es jedoch nicht. Der Verbindungskörper kann in bevorzugten Ausführungen tatsächlich einstückig sein, der Grundkörper umfasst jedoch wenigstens eine Komponente, die relativ zu dem Restkörper bewegbar ist.

Der Grundkörper umfasst ein Gehäuse, eine von dem Gehäuse abragende Kanüle und ein von dem Gehäuse gehaltenes Dichtelement. Das Dichtelement ist vorzugsweise einstückig, kann jedoch durchaus auch aus mehreren Dichtelementstücken zusammengesetzt sein. Eine Unterseite des Gehäuses ist für die Positionierung auf dem Gewebe vorbereitet, beispielsweise indem an der Unterseite des Gehäuses ein Klebepad in üblicher Ausführung angeordnet ist. Die Kanüle kann insbesondere von der Unterseite des Gehäuses abragen. Sie kann beispielsweise aber auch von einer an die Unterseite grenzenden, anderen Seite des Gehäuses abragen. Die Kanüle kann selbst eine Einstechnadel sein. Vorzugsweise ist die Kanüle jedoch flexibel und wird mittels einer Einstechnadel in das Gewebe eingebracht, wobei die Einstechnadel nach dem Einbringen der Kanüle wieder entfernt wird und nur die flexible Kanüle im Gewebe verbleibt. Durch die Positionierung und Befestigung des Grundkörpers auf dem Gewebe wird die eingeführte Kanüle in dem Gewebe gehalten und in diesem Sinne fixiert.

Das Dichtelement weist eine Anschlussseite auf, die dem Verbindungskörper oder genauer gesagt dem Anschlussende der Fluidverbindung des Verbindungskörpers zugewandt ist, wenn der Verbindungskörper mit dem Grundkörper verbunden wird. Die Fluidverbindung des Verbindungskörpers ist an der Anschlussseite oder über die Anschlussseite des Dichtelements mit der Kanüle des Grundkörpers fluidisch verbindbar. Wenn der Grundkörper und der Verbindungskörper miteinander verbunden sind, dichtet das Dichtelement die zwischen dem Verbindungskörper und der Kanüle hergestellte Verbindung nach außen ab, so dass das Fluid ohne Verluste von dem Verbindungskörper zu der Kanüle und/oder in umgekehrter Richtung strömen kann.

Nach der Erfindung umfasst der Grundkörper ferner ein von dem Gehäuse des Grundkörpers bewegbar gelagertes Verschlusselement. Das Verschlusselement ist relativ zu dem Dichtelement so bewegbar, dass es in einer Verschlussposition die Anschlussseite des Dichtelements abdichtet und in einer Freigabeposition für den Fluidkanal des Verbindungskörpers freigibt. Vorzugsweise ist es zwischen der Verschlussposition und der Freigabeposition hin und her bewegbar. Die Bewegung ist eine lineare Verschiebebewegung. Das Verschlusselement ist in seiner Verschlussposition mit einem geeigneten Pressdruck gegen die Anschlussseite des Dichtelements gepresst, so dass ein hermetischer Verschluss der Kanüle zur Umwelt erhalten wird, wenn der Verbindungskörper von dem Grundkörper gelöst ist.

Ein Vorteil des erfindungsgemäßen Verschlusselements ist, dass das Dichtelement kein Septum der herkömmlichen Art sein muss, das von einer Verbindungsnadel durchstochen wird und auch nach mehrmaligem Durchstechen bei herausgezogener Verbindungsnadel die Kanüle des Grundkörpers hermetisch, d.h. steril, nach außen abdichten muss. Die Ausbildung des Dichtelements in solch herkömmlicher Weise und die Ausbildung des Verbindungskörpers mit einer Verbindungsnadel soll jedoch nicht ausgeschlossen sein. Vorteilhafterweise muss aufgrund des erfindungsgemäßen Verschlusselements das Dichtelement jedoch nicht solchen Anforderungen entsprechen. Ein weiterer Vorteil ist, dass der Verbindungskörper keine verletzungsträchtige Verbindungsnadel aufweisen muss. Sein Fluidkanal kann vorteilhaft in einem kurzen Mündungsstutzen ausmünden.

In einer bevorzugten Ausführung weist das Dichtelement sogar einen permanenten Durchlass auf, der sich von der Kanüle bis zu der Anschlussseite des Dichtelements erstreckt. Der Verbindungskörper weist in dieser Ausführung vorzugsweise keine Verbindungsnadel auf. Im verbundenen Zustand von Verbindungskörper und Grundkörper drückt der Verbindungskörper vielmehr mit einem Anschlussende seines Fluidkanals gegen die Anschlussseite des Dichtelements, um die dichte und für medizinische Anwendungen erforderliche sterile Verbindung zwischen dem Fluidkanal des Verbindungskörpers und der Kanüle des Grundkörpers herzustellen. Das Dichtelement wird dementsprechend nicht zum Zweck des Herstellens der Fluidverbindung beschädigt, wie dies bei dem Durchstechen der herkömmlichen Septen zwangsläufig geschieht. Falls das Dichtelement des erfindungsgemäßen Katheterkopfes jedoch ein ebensolches Septum ist und der Verbindungskörper dementsprechend über eine Verbindungsnadel verfügt, wird durch das erfindungsgemäße Verschlusselement in dessen Verschlussposition dennoch eine verbesserte Abdichtung der Kanüle nach außen erreicht.

Zu dem mit einem permanenten Durchlass versehenen Dichtelement ist noch anzumerken, ass der Durchlass sich vorzugsweise durch das Dichtelement hindurch erstreckt. Die Anschlussseite ist an der der Kanüle gegenüberliegenden Seite des Dichtelements gebildet. Anstatt eines Durchlasses kann das Dichtelement für die Herstellung der fluidischen Verbindung von Verbindungskörper und Kanüle einen zu einer Seite offenen Kanal aufweisen. Solch ein einseitig offener Kanal kann auch von dem Gehäuse gebildet werden, wobei in solch einer Ausführung das Dichtelement die offene Seite des Kanals abdichtet.

Das Dichtelement und die Kanüle sind in bevorzugten Ausführungen einstückig gebildet, vorzugsweise aus Kunststoff im Spritzguss. In einer alternativen Ausführung können die Kanüle und das Dichtelement separat gefertigt sein. In solch einer Ausführung kann die Kanüle mit dem Gehäuse gefügt und verbunden sein, beispielsweise indem sie in das Gehäuse dessen Unterseite durchragend eingesetzt ist. Ebenso könnte eine separat gefertigte Kanüle in ein Dichtelement, dieses wenigstens an einer Seite durchragend eingesetzt sein, beispielsweise indem die Kanüle in einem im Dichtelement gebildeten Durchlass eingesetzt ist und dadurch gehalten wird. Ferner ist es durchaus denkbar, die Kanüle in einem Stück mit dem Gehäuse herzustellen, beispielsweise in einem Kunststoffspritzgussverfahren.

Einer unerwünschten Bewegung des Verschlusselements aus der Verschlussposition wirkt bereits die für die Abdichtung der Anschlussseite des Dichtelements erforderliche Presskraft entgegen. Die Presskraft genügt bei entsprechender Konstruktion zwar bereits alleine, um das Verschlusselement ausreichend sicher in der Verschlussposition zu halten, bevorzugt wird es jedoch, wenn das Verschlusselement in der Verschlussposition mit dem Gehäuse des Grundkörpers in einem Blockiereingriff ist, der durch Formschluss verhindert, dass das Verschlusselement sich versehentlich aus der Verschlussposition bewegen kann. Für einen lösbaren, formschlüssigen Blockiereingriff oder einen lösbaren, formschlüssigen und kraftschlüssigen Blockiereingriff weisen das Gehäuse des Grundkörpers und das Verschlusselement je wenigstens ein Blockierelement auf. Das wenigstens eine Blockierelement des Verschlusselements und das wenigstens eine Blockierelement des Gehäuses sind in dem Blockiereingriff vorzugsweise miteinander verrastet. Die Verrastung wird durch Elastizitätskräfte vorzugsweise automatisch bewirkt, wenn das Verschlusselement in seine Verschlussposition gelangt.

Der Verbindungskörper und das Verschlusselement sind vorzugsweise so gestaltet, dass das Verschlusselement durch die Herstellung der Verbindung zwischen dem Grundkörper und dem Verbindungskörper automatisch aus der Verschlussposition in die Freigabeposition bewegt wird. Bevorzugt wird es, wenn der Verbindungskörper das Verschlusselement bei seiner Bewegung, die der Verbindungskörper zum Herstellen der Verbindung relativ zu dem Grundkörper ausführt, einfach mitnimmt. Der Verbindungskörper und das Verschlusselement führen bei dem Verbinden des Verbindungskörpers mit dem Grundkörper relativ zu dem Grundkörper aus der Verschlussposition des Verschlusselements bis in dessen Freigabeposition die gleiche Bewegung aus.

Um die Abdichtung der Anschlussseite des Dichtelements sicher zu gewährleisten, sollte das Verschlusselement automatisch in die Verschlussposition bewegt werden, wenn der Verbindungskörper von dem Grundkörper gelöst wird. In solch einer bevorzugten Ausführung sind das Verschlusselement und der Verbindungskörper entsprechend miteinander gekoppelt, wenn der Grundköper und der Verbindungskörper miteinander verbunden sind. Vorzugsweise bildet sich die Kopplung automatisch, d. h. ohne besondere Handgriffe eigens nur für ihre Herstellung zu erfordern. Die Kopplung ist wie bei der bevorzugt automatisch herbeigeführten Bewegung des Verschlusselements aus der Verschlussposition in die Freigabeposition ebenfalls ein Mitnahmeeingriff, der bewirkt, dass der Verbindungskörper das Verschlusselement mitnimmt, wenn er von dem Grundkörper gelöst wird.

Obgleich eine Kopplung zwischen dem Verbindungskörper und dem Verschlusselement bereits nur für den Zweck vorteilhaft ist, das Verschlusselement automatisch entweder aus der Verschlussposition in die Freigabeposition oder umgekehrt aus der Freigabeposition in die Verschlussposition zu bewegen, wird es bevorzugt, wenn der Verbindungskörper und das Verschlusselement so miteinander gekoppelt sind, dass die Bewegung des Verbindungskörpers relativ zu dem Grundkörper sowohl die Bewegung aus der Verschlussposition als auch die Bewegung in die Verschlussposition bewirkt.

Bezüglich besonders bevorzugter Merkmale und Merkmalskombinationen wird ferner auf die Unteransprüche verwiesen.

Ein Ausführungsbeispiel der Erfindung wird nachfolgend anhand von Figuren erläutert. Es zeigen:
- Figur 1: einen Katheterkopf in einem Längsschnitt entlang einer Symmetrieachse,
- Figur 2: einen Verbindungskörper des Katheterkopfs vor einer Konnektierung,
- Figur 3: einen Grundkörper des Katheterkopfs vor der Konnektierung,
- Figur 4: ein Verschlusselement des Katheterkopfs in einer Einzeldarstellung,
- Figur 5: den Verbindungskörper des Katheterkopfs in einer perspektivischen Sicht auf die Unterseite,
- Figur 6: den Grundkörper in einer Draufsicht, wobei Mitnehmer des Verbindungskörpers vor einem Eingriff mit dem Verschlusselement dargestellt sind,
- Figur 7: den Grundkörper der Figur 6, wobei die Mitnehmer des Verbindungskörpers Mitnehmer des Verschlusselements elastisch abbiegen,
- Figur 8: den Grundkörper der Figuren 6 und 7, wobei die Mitnehmer des Verbindungskörpers relativ zu dem Verschlusselement je in eine Position bewegt wurden, in der die Mitnehmer des Verschlusselements sich wieder elastisch zurückgebogen haben,
- Figur 9: den Grundkörper und den auf dem Grundkörper aufgesetzten Verbindungskörper vor der Konnektierung,
- Figur 10: den Verbindungskörper in einer perspektivischen Sicht und
- Figur 11: den Grundkörper in einer perspektivischen Sicht und einem Schnitt entlang der Symmetrieachse und einem Schnitt rechtwinklig zu der Symmetrieachse.

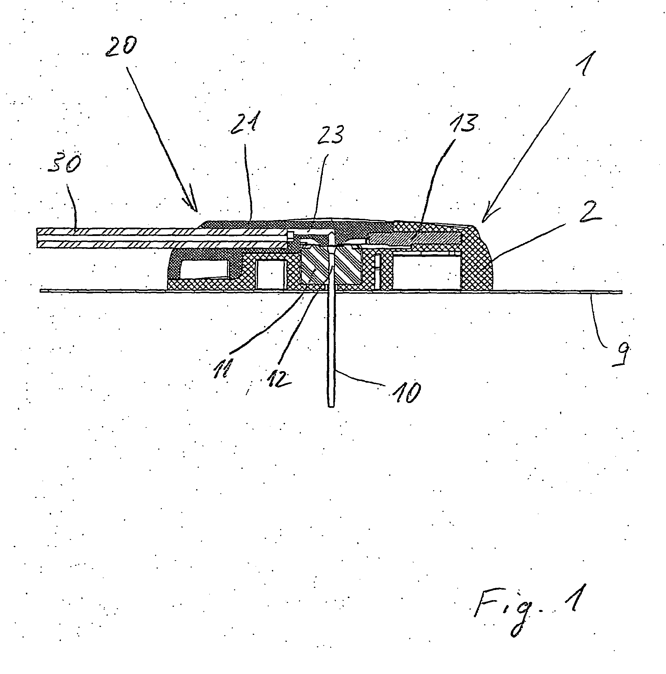

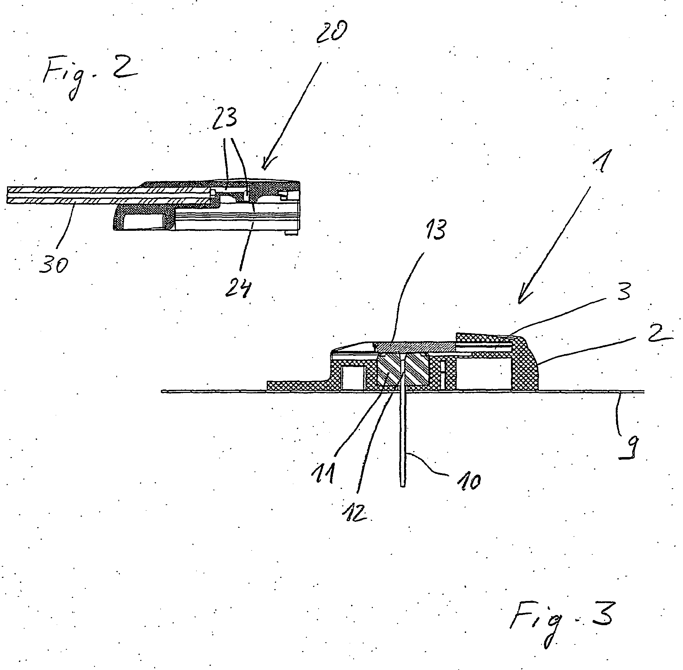

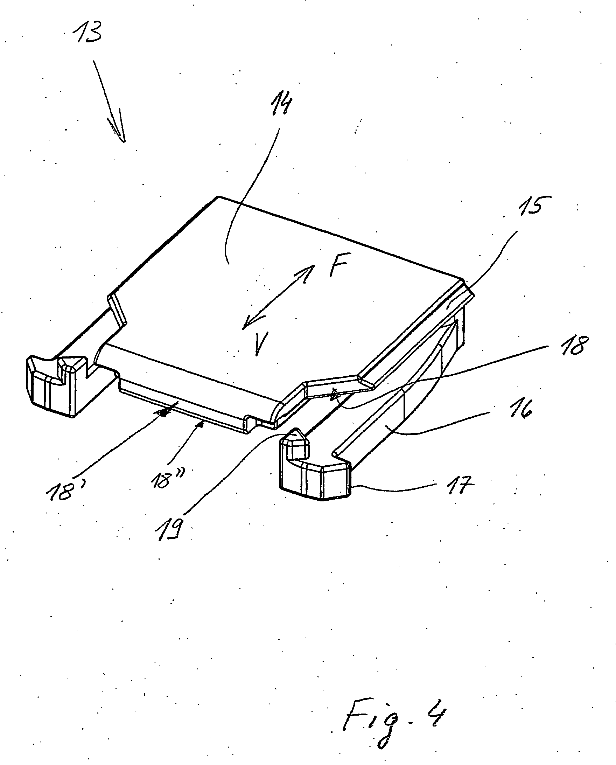

Figur 1 zeigt einen Katheterkopf in einem Längsschnitt. Der Katheterkopf besteht aus einem Grundkörper 1 und einem Verbindungskörper 20, die miteinander lösbar mechanisch und fluidisch konnektiert sind. Der Katheterkopf bildet zusammen mit einem Zuführkatheter 30, der an den Verbindungskörper 20 angeschlossen ist und an seinem freien, nicht dargestellten Ende eine Kupplung für den Anschluss an beispielsweise eine Pumpe aufweist, ein Infusionsset. Das Infusionsset kann insbesondere der Verabreichung eines flüssigen Medikaments dienen. Im Ausführungsbeispiel dient der Katheterkopf der subkutanen Verabreichung von beispielsweise Insulin. Für die Verabreichung weist der Katheterkopf eine Kanüle 10 auf, die unter die Haut in hautnahe Gewebeschichten eingeführt wird. Die Kanüle 10 wird soweit eingeführt, bis der Katheterkopf mit seiner Unterseite auf der Hautoberfläche aufliegt, so dass der Katheterkopf auf der Haut und die Kanüle 10 in dem Gewebe positioniert sind.

Der Grundkörper 1 umfasst ein Gehäuse 2, die Kanüle 10, ein Dichtelement 11 für die Kanüle 10, ein Verschlusselement 13 für das Dichtelement 11 und ein Klebepad 9. Das Gehäuse 2 ist einstückig aus Kunststoff im Spritzguss hergestellt. Das Klebepad 9 ist an einer Unterseite des Gehäuses 2, die gleichzeitig auch die Unterseite des Katheterkopfs bildet, befestigt und dient in bekannter Weise der Befestigung des Katheterkopfs auf der Haut oder in anderen Anwendungen als im Ausführungsbeispiel auf entsprechenden Gewebeoberflächen. Die Kanüle 10 ragt von der Unterseite des Gehäuses 2 ab und durchragt das Klebepad 9. Sie mündet in dem Gehäuse 2 in einen Gehäuseraum, in dem das Dichtelement 11 angeordnet ist.

Das Dichtelement 11 weist einen Durchlass 12 auf, der zu der Unterseite des Gehäuses 2 in die Kanüle 10 mündet. Der Durchlass 12 erstreckt sich durch das Dichtelement 11 hindurch und mündet an einer von der Kanüle 10 abgewandten Anschlussseite des Dichtelements 11, die im Ausführungsbeispiel die von der Kanüle 10 abgewandte Oberseite des Dichtelements 11 ist. Das Dichtelement 11 als solches dichtet die fluidische Verbindung des Verbindungskörpers 20 und der Kanüle 10 ab, indem es das Anschlussende des Verbindungskörpers 20 dicht umgibt und selbst mit seinem permanenten Durchlass 12 das Anschlussende des Verbindungskörpers 20 mit der Kanüle 10 verbindet.

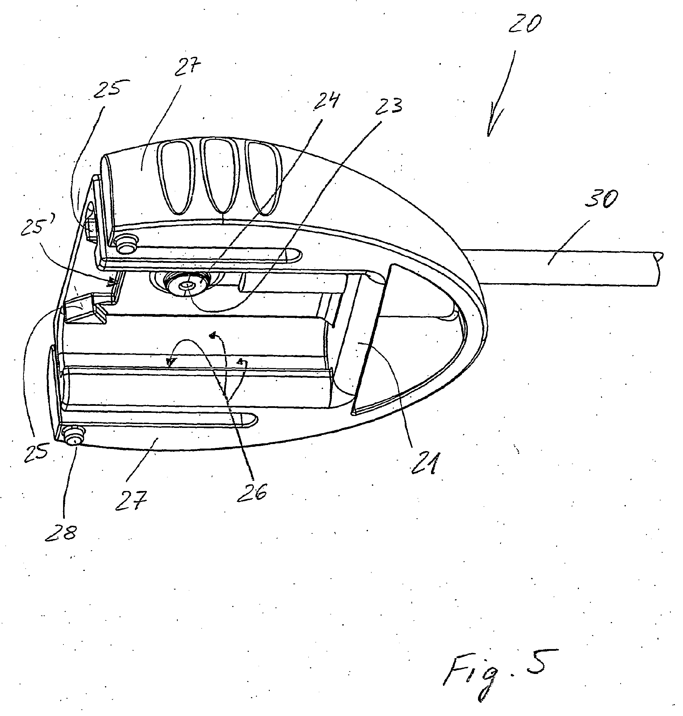

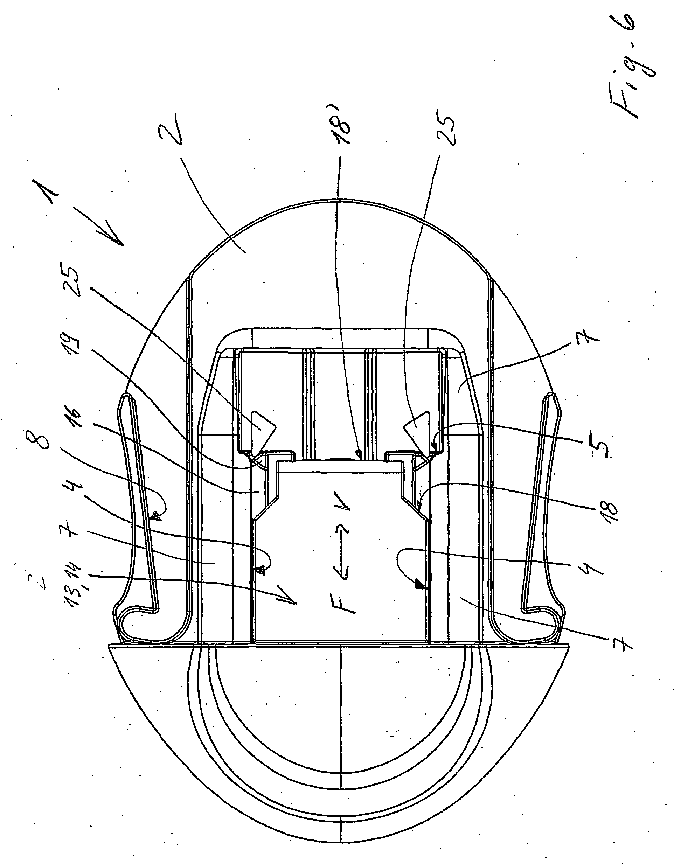

In dem in Figur 1 dargestellten, konnektierten Zustand besteht eine Fluidverbindung zwischen dem Zuführkatheter 30 und der Kanüle 10. Die Fluidverbindung wird durch einen Fluidkanal 23 des Verbindungskörpers 20 und den Durchlass 12 des Dichtelements 11 gebildet. Der Fluidkanal 23 mündet an einer Unterseite des Verbindungskörpers 20, der für die Mündung des Fluidkanals 23 dort einen Mündungsstutzen 24 bildet (Figur 2). Im konnektierten Zustand umschließt der Mündungsstutzen 24 die Mündung des Durchlasses 12 und ist mit einer möglichst kleinen, aber für die gewünschte Dichtwirkung ausreichend großen Presskraft gegen die Anschlussseite des Dichtelements 11 gepresst, um den Fluidkanal 23 dicht mit dem Durchlass 12 zu verbinden.

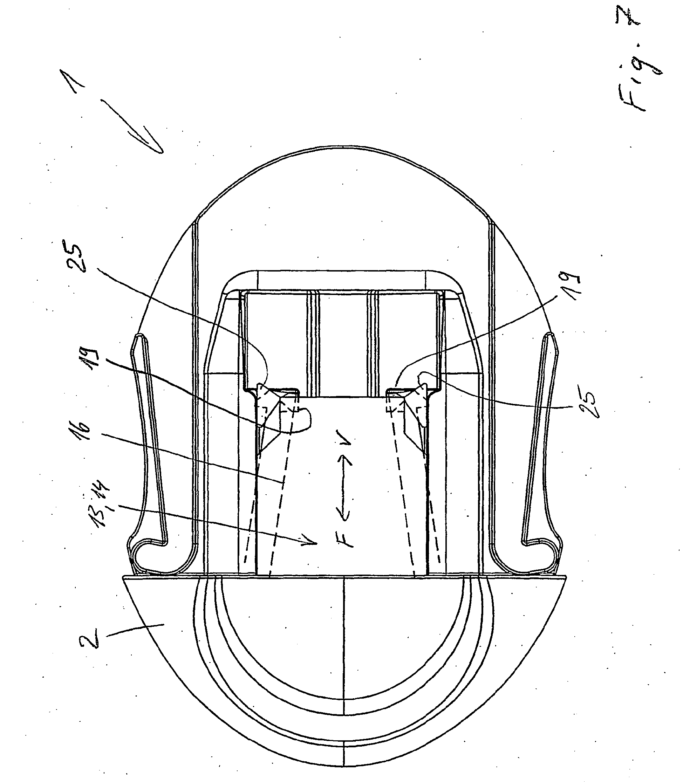

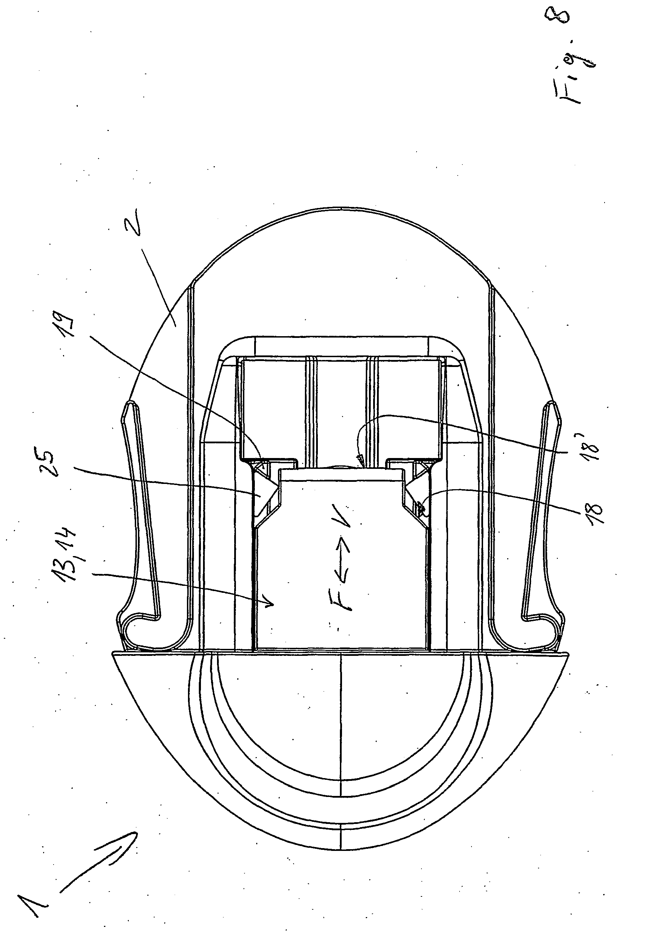

Das Verschlusselement 13 wird von dem Gehäuse 2 des Grundkörpers 1 bewegbar gelagert. Das Verschlusselement 13 ist zwischen einer Freigabeposition, die es in dem konnektierten Zustand (Figur 1) einnimmt, und einer Verschlussposition, die es in dem nicht konnektierten Zustand (Figur 3) einnimmt, hin und her linear verschiebbar. Die Richtung der Bewegbarkeit weist parallel zu der Anschlussseite des Dichtelements 11. In seiner Verschlussposition bildet das Verschlusselement 13 einen Deckel für das Dichtelement 11, der den Durchlass 12 an der Anschlussseite des Dichtelements 11 gegen die Umwelt dicht abschließt, um eine Kontamination der Kanüle 10 zu verhindern, wenn die Kanüle 10 in das Gewebe eingeführt ist. Um die Dichtwirkung zu verstärken, kann das Verschlusselement 13, wie in Figur 1 erkennbar, an seiner der Anschlussseite des Dichtelements 11 zugewandten Unterseite einem erhabenen Bereich aufweisen, mit dem es in der Verschlussposition um die Mündung des Durchlasses 12 herum gegen die Anschlussseite des Dichtelements 11 gepresst ist.

Das Verschlusselement 13 wird bei der Konnektierung des Verbindungskörpers 20 in ein Aufnahmefach 3 (Figur 3) des Gehäuses 2 des Grundkörpers 1 hinein geschoben. Dies wird mittels des Verbindungskörpers 20 bewirkt, der zu diesem Zweck in Richtung der Verschiebbarkeit des Verschlusselements 13 auf das Gehäuse 2 des Grundkörpers 1 aufgeschoben wird. Bei dem Aufschieben drückt der Verbindungskörper 20 das Verschlusselement 13 in die in Figur 1 dargestellte Freigabeposition. Bei der Dekonnektierung wird der Verbindungskörper 20 entgegen seiner Aufschiebrichtung von dem Gehäuse 2 des Grundkörpers 1 abgezogen und nimmt bei seiner Abziehbewegung das Verschlusselement 13 ebenfalls mit bis in die in Figur 3 dargestellte Verschlussposition.

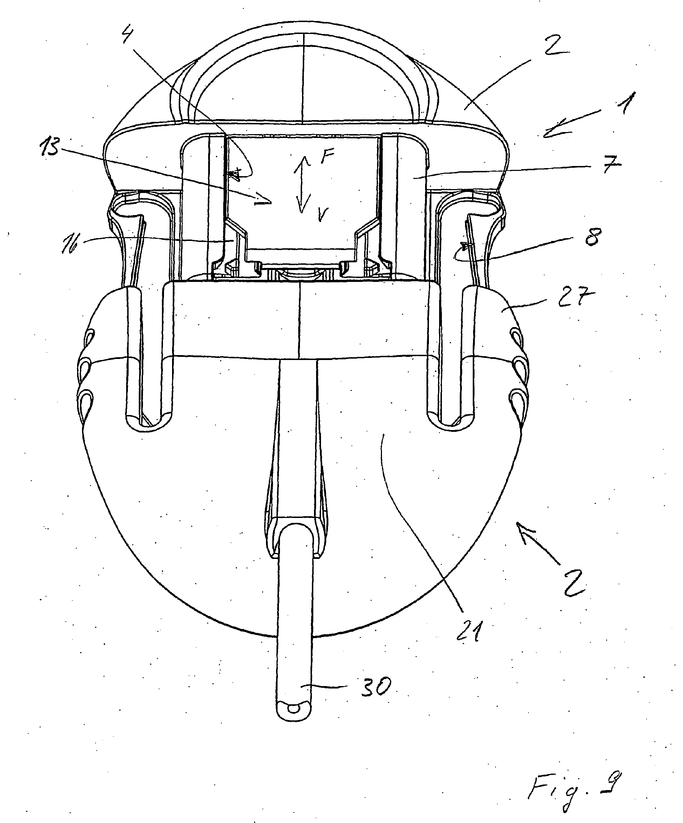

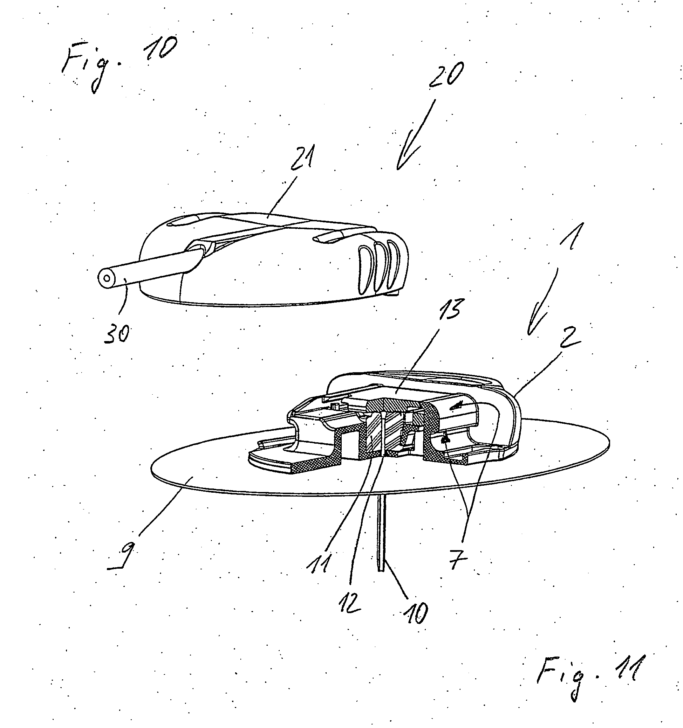

Das Gehäuse 2 und das Verschlusselement 13 bilden eine Linearführung für die Verschiebebewegung des Verschlusselements 13. Das Gehäuse 2 und der Verbindungskörper 20 bilden eine weitere Linearführung für die Aufschieb- und Abziehbewegung des Verbindungskörpers 20 bei der Konnektierung und Dekonnektierung. Die Linearführung für das Verschlusselement 13 wird von den beiden, einander abgewandt gegenüberliegenden Seitenwänden 15 des Verschlusselements 13 (Figur 4) und der jeweils zugewandten von zwei Führungsbahnen 4 (Figur 6) des Gehäuses 2 gebildet. Die Linearführung für den Verbindungskörper 20 wird von Führungsbahnen 7 bildenden Seitenwänden des Gehäuses 2 (Figur 11) und entsprechenden Gegenwandungen 26 des Verbindungskörpers 20 (Figur 5) gebildet.

Beide Linearführungen, nämlich die für das Verschlusselement 13 und die für den Verbindungskörper 20 sind Gleitführungen. Die Gleitführung für den Verbindungskörper 20 ist ferner so geformt, dass der Verbindungskörper 20 sich bei dem Aufschieben auf den Grundkörper 1 an dessen Führungsbahnen 7 zentriert, um das Aufschieben zu erleichtern.

Figur 4 zeigt das Verschlusselement 13 einzeln in einer perspektivischen Sicht auf die Oberseite. Das Verschlusselement 13 weist einen Hauptkörper 14 auf, der den Durchlass 12 des Dichtelements 11 in der Verschlussposition mit seiner Unterseite dicht verschließt. Eingetragen sind in Figur 4 die beiden einander entgegengesetzten Bewegungsrichtungen des Verschlusselements 13, von denen die in die Freigabeposition führende Richtung mit F und die in die Verschlussposition führende Richtung mit V bezeichnet ist.

Von dem in Bezug auf die Richtung F vorderen Ende des Hauptkörpers 14 ragen zwei Schnapparme 16 in Richtung V ab. Die Schnapparme 16 sind quer zu der Richtung der Bewegbarkeit des Verschlusselements 13 biegeelastisch mit dem Hauptkörper 14 verbunden. Die beiden Schnapparme 16 können gegen elastische Rückstellkräfte mit ihren in Bezug auf die Richtung F hinteren Enden aufeinander zu gebogen werden. Figur 4 zeigt die beiden Schnapparme 16 im entspannten Zustand. Jeder der Schnapparme 16 bildet einen Schnapphaken, indem an den freien Enden der Schnapparme 16 je ein nach außen abragender Vorsprung 17 geformt ist. In der Verschlussposition des Verschlusselements 13 verhaken die Schnapphaken, die je von einem der Schnapparme 16 und ihren Vorsprüngen 17 gebildet werden, an dem Gehäuse 2 des Grundkörpers 1, so dass das Verschlusselement 13 in der Verschlussposition gegen eine Bewegung aus der Verschlussposition in die Richtung F auf die Freigabeposition zu gehindert ist. Die Schnapphaken 16, 17 bilden daher Blockierelemente des Verschlusselements 13. Die Blockierelemente 16, 17 wirken mit je einer zurückspringenden Schulter 5 des Gehäuses 2 zusammen. Die Schultern 5 bilden die Blockiergegenelemente des Gehäuses 2 und sind beispielsweise in Figur 6 erkennbar. Bei dem Blockiereingriff zwischen den beiden Blockierelementen 16, 17 und den Blockiergegenelementen 5 handelt es sich um einen Rasteingriff, in den die biegeelastischen Blockierelemente 16, 17 in der Verschlussposition des Verschlusselements 13 vorschnappen und aus der sie gegen ihre elastischen Rückstellkräfte herausbewegt werden können.

An dem in Bezug auf die Richtung F hinteren Ende von jedem der Schnapparme 16 ragt ferner ein Nocken 19 auf. Die beiden Nocken 19 ragen quer zu der Ebene der Biegeelastizität der Schnapparme 16 von deren Oberseiten auf. Die Nocken 19 bilden insbesondere, wie noch erläutert wird, Mitnehmer des Verschlusselements 13, die bei der Dekonnektierung des Verbindungskörpers 20 in einem Mitnahmeeingriff mit entsprechenden Gegenmitnehmern des Verbindungskörpers 20 sind. Die Nocken 19 sind dementsprechend in Richtung V wirksame Mitnehmer des Verschlusselements 13.

Schließlich bildet das Verschlusselement 13 in Richtung F wirksame Mitnehmer 18 und 18', im Ausführungsbeispiel zwei Mitnehmer 18 und einen Mitnehmer 18'. Jeder der Mitnehmer 18 und 18' ist eine von dem Hauptkörper 14 gebildeten Anschlagfläche an der in Richtung V weisenden Rückseite des Hauptkörpers 14. Bei dem Aufschieben des Verbindungskörpers 20 auf den Grundkörper 1 drückt der Verbindungskörper 20 mit entsprechenden Gegenflächen gegen diese Mitnehmer 18 und 18'. Die Gegenflächen bilden bei der Konnektierung die Mitnehmer des Verbindungskörpers 20.

Figur 5 zeigt den Verbindungskörper 20 einzeln in einer perspektivischen Sicht auf seine Unterseite. Erkennbar sind insbesondere die beiden Mitnehmer 25 des Verbindungskörpers 20. Jeder der beiden Mitnehmer 25 ist ein Nocken, der von der Unterseite eines Gehäuses 21 des Verbindungskörpers 20 abragt und bei der Konnektierung und im konnektierten Zustand auf den Grundkörper 1 zuragt. Deutlich erkennbar ist ferner auch der die Mündung des Fluidkanals 23 bildende Mündungsstutzen 24. Der Katheter 30 ist in das Gehäuse 21 eingesteckt und stoffschlüssig mit dem Gehäuse 21 verbunden, beispielsweise verklebt. Der Katheter 30 könnte beispielsweise auch bei einem bevorzugten Spritzgießen des Gehäuses 21 mit dem Material des Gehäuses 21 umgossen werden.

Die Figuren 6, 7 und 8 zeigen in einer Sequenz den Beginn der Konnektierung, d.h. den Beginn des Aufschiebens des Verbindungskörpers 20 auf den Grundkörper 1. Sie sollen insbesondere das Zusammenwirken der Mitnehmer 25 des Verbindungskörpers 20 mit den Mitnehmern 18 und 19 des Versschlusselements 13 demonstrieren. Von dem Verbindungskörper 20 sind lediglich die beiden Mitnehmer 25 dargestellt, um den Blick auf die Schnapparme 16 und die Mitnehmer 18 und 19 des Verschlusselements 13 freizugeben.

In einer ersten Phase der Aufschiebbewegung zentriert sich der Verbindungskörper 20 aufgrund der Form der für das Aufschieben maßgeblichen Linearführung an dem Grundkörper 1, d.h. an dessen Führungsbahnen 7. Sobald der Zentriervorgang abgeschlossen und der Verbindungskörper 20 an den Führungsbahnen 7 eng gleitgeführt ist, gelangen die Mitnehmer 25 mit ihren Vorderseiten in Kontakt mit den Rückseiten der Mitnehmer 19 des Verschlusselements 13. Dieser Zustand ist in Figur 6 dargestellt.

In der sich anschließenden zweiten Phase des Aufschiebens, die in Figur 7 dargestellt ist, gleiten die beiden Mitnehmer 25 an dem beiden Mitnehmer 19 des Verschlusselements 13 entlang. Bei der Gleitbewegung werden die elastischen Schnapparme 16 nach innen aufeinander zu gebogen. Die aufeinander zu gebogenen Schnapparme 16 sind in gestrichelter Linie gezeigt. Sie werden in der Draufsicht der Figur 7 von dem Hauptkörper 14 des Verschlusselements 13 überdeckt. Um das Abgleiten an den Mitnehmern 19 zu erleichtern oder überhaupt erst zu ermöglichen, sind die Vorderseiten der Mitnehmer 25 und die Rückseiten der Mitnehmer 19 rampenförmig geformt, so dass sie aneinander in der Art von schrägen Ebenen abgleiten können. Figur 7 zeigt die Schnapparme 16 bei maximaler Biegung.

Bei dem weiteren Aufschieben des Verbindungskörpers 20 gelangen dessen Mitnehmer 25 vor die Mitnehmer 19 des Verschlusselements 13, wie dies in Figur 8 gezeigt ist. Die Schnapparme 16 sind in dem Zustand der Figur 8 wieder entspannt, d.h. sie haben sich wieder elastisch nach außen voneinander weggebogen.

Bei dem noch weiteren Aufschieben gelangen die Mitnehmer 25 in Kontakt mit den als Anschlagflächen gebildeten Mitnehmern 18 des Verschlusselements 13, so dass in der nun folgenden dritten Phase des Aufschiebens der Verbindungskörper 20 gegen das Verschlusselement 13 drückend dieses in Richtung F bis in die Freigabeposition schiebt. Die Freigabeposition des Verschlusselements 13 ist eine Anschlagposition, d.h. das Verschlusselement 13 ist in der Freigabeposition mit einer in Richtung F weisenden vorderen Anschlagfläche auf Anschlag gegen eine entsprechende Gegenfläche des Gehäuses 2 des Grundkörpers 1. In der Freigabeposition schnappen die Schnapphaken 16, 17 in Ausnehmungen des Gehäuses 2 vor, so dass sie entlastet sind. Auch die Verschlussposition ist eine Anschlagposition, in der das Verschlusselement mit einer bezüglich der Richtung F rückwärtigen, unterhalb von der Mitnehmerfläche 18 gebildeten Fläche 18" auf Anschlag gegen eine entsprechende Gegenfläche des Gehäuses 2 des Grundkörpers 1 liegt.

Wie Figur 8 ferner ebenfalls zeigt, wird auch der Mitnahmeeingriff, der die Mitnahme des Verschlusselements 13 bei der Dekonnektierung des Verbindungskörpers 20 bis in die Verschlussposition bewirkt, bereits bei der Konnektierung hergestellt. Der in Richtung V (Figur 8) wirksame Mitnahmeeingriff besteht nämlich zwischen den Mitnehmern 25 des Verbindungskörpers 20 und den Mitnehmern 19 des Verschlusselements 13, die bei der Konnektierung von den Mitnehmern 25 in Richtung F überschoben wurden. Die von den Mitnehmern 19 und 25 gebildeten Mitnehmerpaare weisen in dem konnektierten Zustand einander zugewandte Flächen auf, an denen sie bei der Mitnahme gegeneinander gedrückt sind. Diese bei der Mitnahme der Dekonnektierung gegeneinander gedrückten Flächen weisen schräg zur Richtung V und sind zueinander parallel. Die Schräge ist so bemessen, dass die Mitnahme sicher bewirkt wird, andererseits der Mitnahmeeingriff sich jedoch selbsttätig löst, wenn das Verschlusselement 13 seine Verschlussposition erreicht hat, aber der Verbindungskörper 20 weiter abgezogen wird. Der Verbindungskörper 20 wird an den Führungen 7 des Grundkörpers 1 über die Verschlussposition des Verschlusselements 13 hinaus noch ein kleines Stück weit in Richtung V geführt. Wenn das Verschlusselement 13 die als Anschlagposition gestaltete Verschlussposition erreicht hat, überschieben die Mitnehmer 25 die Mitnehmer 19 des Verschlusselements 13 in Richtung V, so dass die Schnapparme 16 wieder elastisch aufeinander zu gebogen und schließlich der in Richtung V wirksame Mitnahmeeingriff gelöst wird. Die zurück schnappenden Schnapparme 16 verhaken mit ihren Vorsprüngen 17 wieder hinter den Kraftschultern 5 des Gehäuses 2. Im konnektierten Zustand sind der Grundkörper 1 und der Verbindungskörper 20 miteinander verrastet, um eine versehentlich Dekonnektierung zu verhindern. Die Rastelemente des Grundkörpers 1 werden von zwei Führungsbahnen 8 gebildet, die an ihren in Richtung F vorderen Enden je eine Rastschulter bilden. Die Führungsbahnen 8 sind beispielsweise in den Figuren 8 und 9 deutlich erkennbar. Sie werden in einem hinteren, flachen Bereich des Gehäuses 2 des Grundkörpers 1 je von einer äußeren Seitenwand von zwei in Richtung F sich erstreckenden Ausnehmungen gebildet, die an der Oberseite des flachen Bereichs des Gehäuses 2 zu dem aufschiebenden Verbindungskörper 20 hin offen ist. Die Rastelemente des Verbindungskörpers 20 sind zwei Rastnocken 28 (Figur 5), die von der Unterseite des Verbindungskörpers 20 abragen und bei dem Aufschieben in die jeweils zugeordnete der Ausnehmungen hineinragen. Die Rastelemente 28 des Verbindungskörpers 20 ragen von Flügeln 27 ab, die Betätigungselemente des Verbindungskörpers 20 bilden. Die Flügel 27 ragen biegeelastisch von einem Hauptkörper des Gehäuses 21 ab und können die elastischen Rückstellkräfte aufeinander zu gebogen werden. Die Führungsbahnen 8 für die Rastelemente 28 weisen von ihren hinteren Enden aus gesehen in Richtung F aufeinander zu, beispielsweise einfach schräg, so dass die Flügel 27 durch die an den Führungsbahnen 8 entlang gleitenden Rastelemente 28 elastisch aufeinander zu gebogen werden, wenn der Verbindungskörper 20 auf den Grundkörper 1 aufgeschoben wird. Die Rastschultern der Führungsbahnen 8 sind hinter Kanten der Führungsbahnen 8 gebildet, indem die Führungsbahnen 8 sich an ihren vorderen Enden nach außen plötzlich verbreitern. Bei dem Aufschieben des Verbindungskörpers 8 werden die sich allmählich stärker elastisch aufeinander zu biegenden Flügel 27 daher wieder nach außen entspannt, wenn ihre Rastelemente 28 über die Kante der zugeordneten Führungsbahn 8 hinaus bewegt worden sind. Für die Dekonnektierung drückt der Verwender die beiden Flügel 27 aufeinander zu, so dass deren Rastelemente 28 über die Kanten zurück in die zugeordnete Führungsbahn 8 gebogen wird und deshalb der Verbindungskörper 20 wieder abgezogen werden kann.

Da der Verbindungskörper 20 bei der Konnektierung von dem Grundkörper 1 geführt wird, nämlich mittels der Führungsbahnen 7 in Richtung der Bewegbarkeit des Verschiebelements 13, werden die Rastelemente 28 zwangsweise automatisch an den Führungsbahnen 8 entlang geführt. Der Verwender muss bei der Konnektierung somit nicht einmal den Grundkörper 1 halten, wenn der Grundkörper 1 auf der Haut fixiert ist. Der Verbindungskörper 20 muss auch nicht an den Flügeln 27 gehalten werden, insbesondere müssen die Flügel 27 für die Konnektierung nicht zusammengedrückt werden. Ein Schieben des Verbindungskörpers 20 am Grundkörper 1 reicht aus. Es genügt, wenn der Verwender mit einer Hand den Verbindungskörper 20 hält, beispielsweise am Übergang zwischen dem Verbindungskörper 20 und dem Katheter 30 oder allein an dem Katheter 30. Ferner schnappen die Rastelemente 28 vorzugsweise mit einem hörbaren "Klick" in ihre Rastposition hinter die Kante der jeweils zugeordneten Führungsbahn 8. Der Verwender hört daher, ob der Verbindungskörper 20 sicher mit dem Grundkörper 1 verbunden ist, im Ausführungsbeispiel ob die die Flügel 27 mit dem Grundkörper 1 verrastet sind.

### Bezugszeichenliste

- 1: Grundkörper
- 2: Gehäuse
- 3: Aufnahmefach
- 4: Führungsbahn
- 5: Blockierelement, Schulter
- 6: -
- 7: Führungsbahn
- 8: Führungsbahn
- 9: Klebepad
- 10: Kanüle
- 11: Dichtelement
- 12: Durchlass
- 13: Verschlusselement
- 14: Hauptkörper, Schieber
- 15: Eingriffsglied
- 16: Blockierelement, Schnapper
- 17: Vorsprung, Haken
- 18: Mitnehmer, Anschlag
- 18': Mitnehmer, Anschlag
- 18": Anschlag
- 19: Mitnehmer, Nocken
- 20: Verbindungskörper
- 21: Gehäuse
- 22: -
- 23: Fluidkanal
- 24: Mündungsstutzen
- 25: Mitnehmer, Gegennocken
- 26: Führungsbahn
- 27: Flügel
- 28: Eingriffsglied, Rastelement, Rastnocken
- 29: -
- 30: Katheter

## Patentansprüche

1. Katheterkopf für medizinische und pharmazeutische Anwendungen, der einen auf organischem Gewebe positionierbaren Grundkörper (1) und einen als Fluidanschluss für den Grundkörper (1) dienenden Verbindungskörper (20) umfasst, der einen Fluidkanal (23) aufweist, um den Fluidanschluss zu bilden, wobei der Grundkörper (1) und der Verbindungskörper (20) lösbar miteinander verbunden werden können, der Grundkörper (1) umfassend:
a) ein Gehäuse (2),
b) eine von dem Gehäuse (2) abragende Kanüle (10) zum Einführen in das Gewebe,
c) ein von dem Gehäuse (2) gehaltenes Dichtelement (11) für die Kanüle (10), das eine der Kanüle (10) gegenüberliegende Anschlussseite aufweist, an der oder über die der Fluidkanal (23) des Verbindungskörpers (20) mit der Kanüle (10) fluidisch verbindbar ist,
d) und ein von dem Gehäuse (2) gelagertes Verschlusselement (13), das als Deckel für das Dichtelement (11) ausgebildet ist, der parallel zur Anschlussseite des Dichtelements (11) linear verschiebbar ist, wobei das Verschlusselement (13) in einer Verschlussposition die Anschlussseite des Dichtelements (11) abdichtet und in einer Freigabeposition die Anschlussseite des Dichtelements (11) für den Fluidkanal (23) des Verbindungskörpers (20) freigibt.

2. Katheterkopf nach Anspruch 1, **dadurch gekennzeichnet, dass** das Dichtelement (11) einen Durchlass (12) aufweist, der sich von der Kanüle (10) bis zu der Anschlussseite des Dichtelements (11) erstreckt.

3. Katheterkopf nach dem vorhergehenden Anspruch, **dadurch gekennzeichnet, dass** der Verbindungskörper (20) einen Mündungsstutzen (24) bildet, an dessen freiem Ende der Fluidkanal (23) des Verbindungskörpers (20) in den Durchlass (12) des Dichtelements (11) mündet, wenn der Verbindungskörper (20) mit dem Grundkörper (1) verbunden ist.

4. Katheterkopf nach einem der vorhergehenden Ansprüche, **dadurch gekennzeichnet, dass** ein Blockierelement (5) des Gehäuses (2) und ein Blockierelement (16, 17) des Verschlusselements (13) in der Verschlussposition in einem Blockiereingriff sind, um eine Bewegung des Verschlusselements (13) in die Freigabeposition zu verhindern.

5. Katheterkopf nach dem vorhergehenden Anspruch, **dadurch gekennzeichnet, dass** wenigstens eines der Blockierelemente (5; 16,17) in eine aus dem Blockiereingriff führende Richtung elastisch nachgiebig ist.

6. Katheterkopf nach dem vorhergehenden Anspruch, **dadurch gekennzeichnet, dass** der Verbindungskörper (20) bei dem Verbinden mit dem Grundkörper (1) das elastisch nachgiebige Blockierelement (16, 17) aus dem Blockiereingriff bewegt.

7. Katheterkopf nach einem der vorhergehenden Ansprüche, **dadurch gekennzeichnet, dass** ein Mitnehmer (25, 25') des Verbindungskörpers (20) und ein Mitnehmer (18, 18') des Verschlusselements (13) in einen Mitnahmeeingriff gelangen, wenn der Verbindungskörper (20) mit dem Grundkörper (1) verbunden wird, wobei der Mitnahmeeingriff bewirkt, dass der Verbindungskörper (20) bei einer Bewegung, die er bei dem Verbinden relativ zu dem Grundkörper (1) ausführt, das Verschlusselement (13) bis in die Freigabeposition mitnimmt.

8. Katheterkopf nach einem der vorhergehenden Ansprüche, **dadurch gekennzeichnet, dass** ein Mitnehmer (25) des Verbindungskörpers (20) und ein Mitnehmer (19) des Verschlusselements (13) in einem Mitnahmeeingriff sind, wenn der Verbindungskörper (20) von dem Grundkörper (1) gelöst wird, wobei der Mitnahmeeingriff bewirkt, dass der Verbindungskörper (20) bei einer Bewegung, die er bei dem Lösen relativ zu dem Grundkörper (1) ausführt, das Verschlusselement (13) bis in die Verschlussposition mitnimmt.

9. Katheterkopf nach dem vorhergehenden Anspruch, **dadurch gekennzeichnet, dass** wenigstens einer (19) der Mitnehmer (19, 25) an einem elastisch nachgiebigen Schnapper (16) gebildet ist und die Mitnehmer (19, 25) durch ein elastisches Nachgeben des Schnappers (16) in den Mitnahmeeingriff gelangt, vorzugsweise bereits wenn der Verbindungskörper (20) mit dem Grundkörper (1) verbunden wird.

10. Katheterkopf nach einem der zwei vorhergehenden Ansprüche, **dadurch gekennzeichnet, dass** der Mitnehmer (25) des Verbindungskörpers (20) auch den Mitnehmer (25) des Verbindungskörpers (20) nach Anspruch 7 bildet.

11. Katheterkopf nach einem der vorhergehenden Ansprüche, **dadurch gekennzeichnet, dass** die Verschlussposition des Verschlusselements (13) eine Anschlagposition ist.

12. Katheterkopf nach einem der vorhergehenden Ansprüche, **dadurch gekennzeichnet, dass** die Freigabeposition des Verschlusselements (13) eine Anschlagposition ist.

13. Katheterkopf nach einem der vorhergehenden Ansprüche, **dadurch gekennzeichnet, dass** der Verbindungskörper (20) und der Grundkörper (1) translativ aneinander geführt sind, wenn sie miteinander verbunden und wenn sie voneinander gelöst werden.

14. Katheterkopf nach dem vorhergehenden Anspruch, **dadurch gekennzeichnet, dass** der Verbindungskörper (20) und der Grundkörper (1) aneinander geführt werden und dass sie sich aneinander zentrieren, wenn sie miteinander verbunden werden.

15. Katheterkopf nach einem der vorhergehenden Ansprüche, **dadurch gekennzeichnet, dass** der Verbindungskörper (20) und das Verschlusselement (13) zwischen der Verschlussposition und der Freigabeposition des Verschlusselements (13) von dem Grundkörper (1) in die gleiche Richtung bewegbar geführt werden.

16. Katheterkopf nach einem der vorhergehenden Ansprüche, **dadurch gekennzeichnet, dass** der Verbindungskörper (20) wenigstens einen biegeelastischen Flügel (27) aufweist, der biegeelastisch mit dem Grundkörper (1) verrastet, wenn der Fluidkanal (23) des Verbindungskörpers (20) mit der Kanüle (10) verbunden ist.

17. Katheterkopf nach dem vorhergehenden Anspruch, **dadurch gekennzeichnet, dass** der Grundkörper (1) und der Flügel (27) eine Führungsbahn (8) und ein Eingriffsglied (28) bilden, das bei dem Verbinden des Verbindungskörpers (20) mit dem Grundkörper (1) an der Führungsbahn (8) geführt wird, wobei das Eingriffsglied (28) quer zu der Richtung der Biegeelastizität des Flügels (27) von dem Flügel (27) oder von dem Gehäuse des Grundkörpers abragt.

18. Katheterkopf nach einem der zwei vorhergehenden Ansprüche, **dadurch gekennzeichnet, dass** die Führungsbahn (8) eine Rastschulter bildet, hinter der das Eingriffsglied (28) verrastet, wenn der Fluidkanal (23) des Verbindungskörpers (20) mit der Kanüle (10) verbunden ist.

## Claims

1. Catheter head for medical and pharmaceutical applications, comprising a base body (1) which can be positioned on organic tissue, and a connecting body (20) which serves as fluid connector for the base body (1) and has a fluid channel (23) for forming the fluid connector, the base body (1) and the connecting body (20) being able to be connected to one another in a detachable manner, the base body (1) comprising:
a) a housing (2),
b) a cannula (10) protruding from the housing (2) and serving for insertion into the tissue,
c) a seal element (11) for the cannula (10) which is held by the housing (2) and which has a connector face opposite the cannula (10) on which or via which the fluid channel (23) of the connecting body (20) can be placed in fluidic communication with the cannula (10),
d) and a closure element (13) which is supported by the housing (2), is configured as a cover for the seal element (11) and is linearly displaceable parallel to the seal element (11) the closure element (13), in a closure position, sealing off the connector face of the seal element (11) and, in an open position, opening the connector face of the seal element (11) for the fluid channel (23) of the connecting body (20).

2. Catheter head according to Claim 1, **characterized in that** the seal element (11) has a passage (12) extending from the cannula (10) as far as the connector face of the seal element (11).

3. Catheter head according to the preceding claim, **characterized in that** the connecting body (20) forms an outlet nozzle (24) at whose free end the fluid channel (23) of the connecting body (20) opens into the passage (12) of the seal element (11) when the connecting body (20) is connected to the base body (1).

4. Catheter head according to one of the preceding claims, **characterized in that** a blocking element (5) of the housing (2) and a blocking element (16, 17) of the closure element (13) are in a blocking engagement in the closure position, in order to prevent a movement of the closure element (13) into the open position.

5. Catheter head according to the preceding claim, **characterized in that** at least one of the blocking elements (5; 16, 17) is elastically resilient in a direction out from the blocking engagement.

6. Catheter head according to the preceding claim, **characterized in that** the connecting body (20), upon connection to the base body (1), moves the elastically resilient blocking element (16, 17) out from the blocking engagement.

7. Catheter head according to one of the preceding claims, **characterized in that** a driver (25, 25') of the connecting body (20) and a driver (18, 18') of the closure element (13) come into driving engagement when the connecting body (20) is connected to the base body (1), the driving engagement having the effect that the connecting body (20), in a movement it makes relative to the base body (1) upon connection, drives the closure element (13) into the open position.

8. Catheter head according to one of the preceding claims, **characterized in that** a driver (25) of the connecting body (20) and a driver (19) of the closure element (13) are in driving engagement when the connecting body (20) is detached from the base body (1), the driving engagement having the effect that the connecting body (20), in a movement it makes relative to the base body (1) upon detachment, drives the closure element (13) into the closure position.

9. Catheter head according to the preceding claim, **characterized in that** at least one (19) of the drivers (19, 25) is formed on an elastically resilient snap-in catch (16) and the drivers (19, 25) come into driving engagement via the elastic resilience of the snap-in catch (16), preferably already when the connecting body (20) is connected to the base body (1).

10. Catheter head according to one of the two preceding claims, **characterized in that** the driver (25) of the connecting body (20) also forms the driver (25) of the connecting body (20) in Claim 7.

11. Catheter head according to one of the preceding claims, **characterized in that** the closure position of the closure element (13) is an abutment position.

12. Catheter head according to one of the preceding claims, **characterized in that** the open position of the closure element (13) is an abutment position.

13. Catheter head according to one of the preceding claims, **characterized in that** the connecting body (20) and the base body (1) are guided in translation on one another when they are being connected to one another and when they are being detached from one another.

14. Catheter head according to the preceding claim, **characterized in that** the connecting body (20) and the base body (1) are guided on one another and **in that** they are centered on one another when they are being connected to one another.

15. Catheter head according to one of the preceding claims, **characterized in that**, between the closure position and the open position of the closure element (13), the connecting body (20) and the closure element (13) are guided movably in the same direction from the base body (1).

16. Catheter head according to one of the preceding claims, **characterized in that** the connecting body (20) has at least one flexurally elastic wing (27) which locks in a flexurally elastic manner with the base body (1) when the fluid channel (23) of the connecting body (20) is connected to the cannula (10).

17. Catheter head according to the preceding claim, **characterized in that** the base body (1) and the wing (27) form a guide track (8) and an engagement member (28) which, upon connection of the connecting body (20) to the base body (1), is guided on the guide track (8), the engagement member (28) protruding from the wing (27) or from the housing of the base body transversely with respect to the direction of the flexural elasticity of the wing (27).

18. Catheter head according to one of the two preceding claims, **characterized in that** the guide track (8) forms a locking shoulder behind which the engagement member (28) locks when the fluid channel (23) of the connecting body (20) is connected to the cannula (10).

## Revendications

1. Tête de cathéter pour applications médicales et pharmaceutiques, qui comprend un corps de base (1) pouvant être positionné sur un tissu organique, et un corps de connexion (20) servant de raccordement fluidique pour le corps de base (1), qui présente un canal de fluide (23) afin de former le raccordement fluidique, le corps de base (1) et le corps de connexion (20) pouvant être connectés l'un à l'autre de manière détachable, le corps de base (1) comprenant :
a) un boîtier (2),
b) une canule (10) dépassant du boîtier (2) destinée à être introduite dans le tissu,
c) un élément d'étanchéité (11) maintenu par le boîtier (2) pour la canule (10), qui présente un côté de raccordement opposé à la canule (10), au niveau duquel ou par le biais duquel le canal de fluide (23) du corps de connexion (20) peut être connecté fluidiquement à la canule (10),
d) et un élément de fermeture (13) porté par le boîtier (2), qui est réalisé sous forme de couvercle pour l'élément d'étanchéité (11), qui peut coulisser linéairement parallèlement au côté de raccordement de l'élément d'étanchéité (11), l'élément de fermeture (13) fermant hermétiquement le côté de raccordement de l'élément d'étanchéité (11) dans une position de fermeture, et libérant le côté de raccordement de l'élément d'étanchéité (11) pour le canal de fluide (23) du corps de connexion (20) dans une position de libération.

2. Tête de cathéter selon la revendication 1, **caractérisée en ce que** l'élément d'étanchéité (11) présente un passage (12) qui s'étend depuis la canule (10) jusqu'au côté de raccordement de l'élément d'étanchéité (11).

3. Tête de cathéter selon la revendication précédente, **caractérisée en ce que** le corps de connexion (20) forme un embout (24) à l'extrémité libre duquel le canal de fluide (23) du corps de connexion (20) débouche dans le passage (12) de l'élément d'étanchéité (11) lorsque le corps de connexion (20) est connecté au corps de base (1).

4. Tête de cathéter selon l'une quelconque des revendications précédentes, **caractérisée en ce qu'**un élément de blocage (5) du boîtier (2) et un élément de blocage (16, 17) de l'élément de fermeture (13) sont en engagement de blocage dans la position de fermeture, afin d'empêcher un mouvement de l'élément de fermeture (13) dans la position de libération.

5. Tête de cathéter selon la revendication précédente, **caractérisée en ce qu'**au moins l'un des éléments de blocage (5 ; 16, 17) est flexible élastiquement dans une direction sortant de l'engagement de blocage.

6. Tête de cathéter selon la revendication précédente, **caractérisée en ce que** le corps de connexion (20) déplace l'élément de blocage flexible élastiquement (16, 17) hors de l'engagement de blocage lors de la connexion avec le corps de base (1).

7. Tête de cathéter selon l'une quelconque des revendications précédentes, **caractérisée en ce qu'**un organe d'entraînement (25, 25') du corps de connexion (20) et un organe d'entraînement (18, 18') de l'élément de fermeture (13) parviennent en engagement d'entraînement lorsque le corps de connexion (20) est connecté au corps de base (1), l'engagement d'entraînement faisant en sorte que le corps de connexion (20) entraîne l'élément de fermeture (13) jusque dans la position de libération dans le cas d'un déplacement qu'il effectue par rapport au corps de base (1) lors de la connexion.

8. Tête de cathéter selon l'une quelconque des revendications précédentes, **caractérisée en ce qu'**un organe d'entraînement (25) du corps de connexion (20) et un organe d'entraînement (19) de l'élément de fermeture (13) sont en engagement d'entraînement lorsque le corps de connexion (20) est détaché du corps de base (1), l'engagement d'entraînement faisant en sorte que le corps de connexion (20) entraîne l'élément de fermeture (13) jusque dans la position de fermeture dans le cas d'un déplacement qu'il effectue par rapport au corps de base (1) lors du relâchement.

9. Tête de cathéter selon la revendication précédente, **caractérisée en ce qu'**au moins l'un (19) des organes d'entraînement (19, 25) est formé sur un accouplement à déclic flexible élastiquement (16) et l'organe d'entraînement (19, 25) parvient par une flexion élastique de l'accouplement à déclic (16) en engagement d'entraînement, de préférence déjà lorsque le corps de connexion (20) est connecté au corps de base (1).

10. Tête de cathéter selon l'une quelconque des deux revendications précédentes, **caractérisée en ce que** l'organe d'entraînement (25) du corps de connexion (20) forme également l'organe d'entraînement (25) du corps de connexion (20) selon la revendication 7.

11. Tête de cathéter selon l'une quelconque des revendications précédentes, **caractérisée en ce que** la position de fermeture de l'élément de fermeture (13) est une position de butée.

12. Tête de cathéter selon l'une quelconque des revendications précédentes, **caractérisée en ce que** la position de libération de l'élément de fermeture (13) est une position de butée.

13. Tête de cathéter selon l'une quelconque des revendications précédentes, **caractérisée en ce que** le corps de connexion (20) et le corps de base (1) sont guidés en translation l'un contre l'autre, lorsqu'ils sont connectés ensemble et qu'ils sont détachés l'un de l'autre.

14. Tête de cathéter selon la revendication précédente, **caractérisée en ce que** le corps de connexion (20) et le corps de base (1) sont guidés l'un contre l'autre et **en ce qu'**ils se centrent l'un contre l'autre lorsqu'ils sont connectés ensemble.

15. Tête de cathéter selon l'une quelconque des revendications précédentes, **caractérisée en ce que** le corps de connexion (20) et l'élément de fermeture (13) sont guidés de manière déplaçable dans la même direction par le corps de base (1) entre la position de fermeture et la position de libération de l'élément de fermeture (13).

16. Tête de cathéter selon l'une quelconque des revendications précédentes, **caractérisée en ce que** le corps de connexion (20) présente au moins une aile (27) flexible élastique, qui s'encliquète de manière flexible et élastique avec le corps de base (1) lorsque le canal de fluide (23) du corps de connexion (20) est connecté à la canule (10).

17. Tête de cathéter selon la revendication précédente, **caractérisée en ce que** le corps de base (1) et l'aile (27) forment une piste de guidage (8) et un organe d'engagement (28), qui est guidé lors de la connexion du corps de connexion (20) au corps de base (1) sur la piste de guidage (8), l'organe d'engagement (28) dépassant transversalement de l'aile (27) ou du boîtier du corps de base par rapport à la direction de l'élasticité en flexion de l'aile (27).

18. Tête de cathéter selon l'une quelconque des deux revendications précédentes, **caractérisée en ce que** la piste de guidage (8) forme un épaulement d'encliquetage derrière lequel l'organe d'engagement (28) s'encliquète, lorsque le canal de fluide (23) du corps de connexion (20) est connecté à la canule (10).
